# EUROPEAN PATENT SPECIFICATION

(11) **EP 4 129 333 B1**
(45) Date of publication and mention of the grant of the patent: **02.12.2026**
(21) Application number: 21776170.9
(22) Date of filing: 26.03.2021
(51) Int. Cl.: A61K 39/395, A61P 35/00, A61P 35/02, A61K 47/68, A61K 38/16, A61K 41/00, A61K 31/409, A61K 38/45, A61K 38/47, A61K 47/64, C07K 16/28, C07K 16/32

(54) **SAPORIN-CONJUGATED ANTIBODY OR ANTIBODY FRAGMENT IN COMBINATION WITH TALAPORFIN SODIUM FOR DESTROYING TUMOR CELLS**
ANTIKÖRPER ODER ANTIKÖRPERFRAGMENT KONJUGIERT MIT SAPORIN IN KOMBINATION MIT TALAPORFIN-NATRIUM ZUR ZERSTÖRUNG VON TUMORZELLEN
ANTICORPS OU FRAGMENT D'ANTICORPS CONJUGUÉ À LA SAPORINE EN COMBINAISON AVEC DU TALAPORFINE SODIUM POUR DÉTRUIRE DES CELLULES TUMORALES

(30) Priority: 27.03.2020 JP 2020057561
(43) Date of publication of application: 08.02.2023
(73) Proprietor: PhotoQ3 Inc., Tokyo 102-0083 (JP)
(72) Inventor: TODA Naoko, Tokyo 102-0084 (JP); SUDO Yukio, Tokyo 102-0084 (JP); HAMAKUBO Takao, Tokyo 102-0084 (JP)
(74) Representative: Hoffmann Eitle
(86) International application number: PCT/JP2021/012922
(87) International publication number: WO 2021/193928

(56) References cited:
- WO-A1-2018/156725
- WO-A1-2018/195302
- WO-A2-2006/132670
- CN-A- 110 229 323
- US-B2- 7 994 135
- BASKARAN RENGARAJAN, LEE JUNGHAN, YANG SU-GEUN: "Clinical development of photodynamic agents and therapeutic applications", BIOMATER RES, vol. 22, no. 25, 2018, pages 1 - 8, XP055861893, ISSN: 1226-4601
- GILABERT-ORIOL, R. ET AL.: "Modified Trastuzumab and Cetuximab Mediate Efficient Toxin Delivery While Retaining Antibody-Dependent Cell -Mediated Cytotoxicity in Target Cells", MOL PHARM, vol. 10, 2013, pages 4347 - 57, XP055677139, ISSN: 1543-8384, DOI: 10.1021/mp400444q

## Description

### Technical Field

The present invention relates to a medicament for killing tumor cells, comprising a conjugate of saporin and an antibody or an antibody fragment that binds to a target substance on the surface of tumor cells, and Talaporfin Sodium.

### Background Art

In Japan, approximately 370,000 people die of cancer each year, and cancer has continuously been a leading cause of death since 1981. To date, a large number of therapeutic methods for cancer have been developed inside and outside of the country. However, a specific remedy for cancer has not yet been developed.

What is called, small molecule anticancer agents (cancer chemotherapy) have been developed. However, the medicinal effects of these anticancer agents are not strong enough, and further, severe side effects cause trouble to patients. Thus, it cannot be said that these anticancer agents are desirable medicaments. Since antibody drugs are characterized in that the antibody drugs have strong specificity to cancer, strong side effects found in the small molecule anticancer agents can be alleviated, and thus, such antibody drugs can be broadly used. However, there are yet only a small number of antibody drugs effective for solid cancer. In order to reinforce medicinal effects, ADC (antibody-drug conjugate) has been developed, but such ADC has not yet been satisfactory, including its toxicity.

Moreover, an immunotherapeutic antibody (checkpoint inhibitor) used as a novel antibody drug exhibits strong medicinal effects against a wide range of cancer species based on its novel mechanism. However, it has been found that the number of patients who receive the effects of such an immunotherapeutic antibody is not necessarily large, and that, in some cases, the patients have severe side effects to such an extent that they lead to death.

On the other hand, on the basis of an idea that the therapeutic site is limited and side effects are thereby reduced, PDT (Photo Dynamic Therapy) has been developed. PDT is a therapeutic method by which a light having a certain wavelength that activates photosensitizing dyes gathering in a tumor site is applied to an affected area to treat it. PDT shows certain effects against lung cancer, etc., but it cannot be said that satisfactory medicinal effects are obtained from this therapeutic method.

Doronina et al. (US 7,994,135) relates to monomethylvaline compounds capable of conjugation to ligands. Andrews et al. (WO 2018/156725 A1) relates to methods and composition for inhibiting tumor growth and enhancing immune response to tumors. Doronina et al. (WO 2006/132670 A2) relates to auristatins having an aminobenzoic acid unit at the N-terminus. Guo et al. (WO 2018/195302 A1) relates to anti-VTCN1 antibodies and antibody drug conjugates. Baskaran et al. (Biomater Res, 2018, 22(25): 1-8) relates to clinical development of photodynamic agents and therapeutic applications. Gilabert-Oriol et al. (Mol Pharm, 2013, 10: 4347-57) relates to modified Trastuzumab and Cetuximab mediating toxin delivery while retaining antibody-dependent cell - mediated cytotoxicity in target cells.

### Summary of invention

The present invention relates to a medicament for use in a method of killing tumor cells in a subject, comprising: a conjugate of saporin and an antibody or an antibody fragment that binds to a target substance on the surface of tumor cells; and Talaporfin Sodium, wherein the medicament is for use in combination with irradiation of wavelength effective for activating the Talaporfin Sodium.

The invention is further defined in the claims.

### Means for Solving the Objects

PDT is a method for destroying tumor cells by accumulating sensitizing dyes to tumor tissues and then applying a light to the tumor cells. As such sensitizing dyes used in PDT, sensitizing dyes having high water-solubility and a high property of accumulating in tumor, such as Talaporfin Sodium, have been known, and sensitizing dyes have already been used in the treatment of lung cancer and the like. PDT is a low-invasive therapeutic method, but this method is disadvantageous in that the medicinal effects thereof are not necessarily satisfactory.

PCI (Photochemical Internalization) is a method for destroying an endosomal membrane by accumulating photosensitizers to an endosomal membrane and then applying a light to the endosomal membrane. It is considered that, according to PCI, anticancer agents or immunotoxins enclosed in the endosomal membrane are released into the cytoplasm, so that tumor cells are destroyed. For the purpose of accumulating photosensitizers to the endosomal membrane, the dyes used in PCI are different from those used in PDT, and amphipathic photosensitizers such as sulfonated tetraphenylchlorin (TPCS2a) or aluminum phthalocyanine (AlPcS2a) are used.

However, such amphipathic sulfonated tetraphenylchlorin (TPCS2a) or aluminum phthalocyanine (AlPcS2a) is disadvantageous in that, for example, (1) neurotoxicity is concerned, in that (2) the photosensitizers accumulate in cell membranes other than the endosomal membrane and cause cytotoxicity, and in that (3) the photosensitizers have a low property of accumulating in tumor and cause non-specific side effects.

The present inventors have conducted intensive studies directed towards solving the previous problems. As a result, the present inventors have found that highly water-soluble Talaporfin Sodium has such unpredictable effects that it increases the permeability of the endosomal membrane even at a low concentration. Moreover, the present inventors have also found that, by combining a conjugate of a substance that binds to a target substance on the surface of tumor cells and a cytotoxin, with Talaporfin Sodium cytotoxicity and tumor specificity can be significantly reinforced, thereby completing the present invention.

### Brief Description of Drawings

[Fig. 1] Fig. 1 shows cell viability in an A431 cytotoxicity assay.
[Fig. 2] Fig. 2 shows cell viability in an A549 cytotoxicity assay.
[Fig. 3] Fig. 3 shows cell viability in an MKN7 cytotoxicity assay.
[Fig. 4] Fig. 4 shows cell viability in an MDA-MB-453 cytotoxicity assay.
[Fig. 5] Fig. 5 shows cell viability in an A431/PEP-T cytotoxicity assay.
[Fig. 6] Fig. 6 shows cell viability in an A431/Verteporfin cytotoxicity assay.

### Embodiment of Carrying out the Invention

The present inventors have studied a method for treating a tumor, which has strong medicinal effects and few side effects, and as a result, they have conceived that the techniques PDT and PCI that topically enhance medicinal effects as a result of light irradiation have potential. However, PDT has been disadvantageous in terms of weak medicinal effects, whereas PCI has not been satisfactory in terms of both medicinal effects and toxicity.

Hence, the present inventors have conducted intensive studies, and as a result, they have found for the first time that a water-soluble sensitizing dye, namely, Talaporfin improves the permeability of the endosome by light irradiation. By combining this "dye," "a conjugate of a substance that binds to a target substance on the surface of tumor cells and a cytotoxin," and "light irradiation to a tumor," the present inventors have discovered a therapeutic method having strong medicinal effects and few side effects, thereby completing the present invention.

Specifically, in the present invention, a conjugate of a substance that binds to a target substance on the surface of tumor cells and a cytotoxin binds to a tumor, and it is then enclosed in the endosome. It is conceived that a light is then applied to Talaporfin Sodium which has been added separately (or simultaneously), so that an immunotoxin (or a decomposed product thereof) in the endosome is released into the cytoplasm, and thereby, the tumor cells can be killed.

Talaporfin Sodium is advantageous in that it is water-soluble, and so, differing from amphipathic sulfonated tetraphenylchlorin (TPCS2a) or aluminum phthalocyanine (AlPcS2a), this substance is highly unlikely to cause side effects as it is not directed to localize on the membrane. Furthermore, since the absorption wavelength of these substances is 664 nm, which is not overlapped with the absorption wavelength of hemoglobin, the depth reached by light is deep.

### < Embodiments regarding Method for Killing Cells >

The method for killing tumor cells of the present invention may include an embodiment in which tumor cells are irradiated with (3) a light having a wavelength for activating Talaporfin Sodium in the coexistence of (1) a conjugate of a substance that binds to a target substance on the surface of tumor cells and a cytotoxin, and (2) the Talaporfin Sodium.

As a further specific embodiment, the killing of tumor cells can also be achieved by administering (1) a conjugate of a substance that binds to a target substance on the surface of tumor cells and a cytotoxin to a subject, then administering thereto (2) Talaporfin Sodium and then irradiating the cells with (3) a light having a wavelength for activating the Talaporfin Sodium.

In addition, as another embodiment, the killing of tumor cells can also be achieved by administering (1) Talaporfin Sodium to a subject, then administering thereto (2) a conjugate of a substance that binds to a target substance on the surface of tumor cells and a cytotoxin, and then irradiating the cells with (3) a light having a wavelength for activating the Talaporfin Sodium

Moreover, as another embodiment, the killing of tumor cells can also be achieved by simultaneously administering (1) a conjugate of a substance that binds to a target substance on the surface of tumor cells and a cytotoxin and (2) Talaporfin Sodium to a subject, and then irradiating the cells with (3) a light having a wavelength for activating the Talaporfin Sodium.

### < Photosensitizing Dye >

In the present invention, Talaporfin Sodium is used as a sensitizer.

Talaporfin Sodium is a sensitizing dye used in PDT, which is also referred to as Laserphyrin, NPe6, or mono-aspartyl chlorin e6.

### < Substance that Binds to Target Substance on Surface of Tumor Cells >

The substance that binds to a target substance on the surface of tumor cells an antibody or an antibody fragment.

It is possible to use an antibody that specifically binds to a target substance on the surface of tumor cells (for example, a protein, such as Epidermal Growth Factor Receptor (EGFR, ERBB 1, ERBB2, ERBB3, or ERBB4), Mesothelin, Ephrin type-A receptor 2 (EphA2), Glypican 3 (GPC3), Cadhelin 17 (CDH17), Cadherin 3 (CDH3), or Roundabout homolog 1 (Robo1)).

The type of the antibody used in the present invention is not particularly limited, and examples of the present antibody may include a mouse antibody, a human antibody, a rat antibody, a rabbit antibody, a sheep antibody, a camel antibody, an avian antibody, and a genetically modified antibody that is artificially modified for the purpose of reducing xenoantigenicity against a human, such as a chimeric antibody or a humanized antibody. Such a genetically modified antibody can be produced by applying a known method. The chimeric antibody is an antibody consisting of the heavy chain and light chain variable regions of a mammalian antibody other than a human antibody, such as a mouse antibody, and the heavy chain and light chain constant regions of a human antibody. The chimeric antibody can be obtained by ligating DNA encoding the variable region of a mouse antibody to DNA encoding the constant region of a human antibody, then incorporating the ligate into an expression vector, and then introducing the expression vector into a host, so that the host is allowed to generate the antibody. The humanized antibody is obtained by transplanting the complementarity determining region (CDR) of a mammalian antibody other than a human antibody, such as a mouse antibody, into the complementarity determining region of a human antibody. A common gene recombination method therefor has been known. Specifically, a DNA sequence designed to ligate the CDR of a mouse antibody to the framework region (FR) of a human antibody is synthesized from several oligonucleotides that have been produced such that they have an overlapping portion at the terminal portions thereof according to a PCR method. The obtained DNA is ligated to DNA encoding the constant region of a human antibody, and the ligate is then incorporated into an expression vector, which is then introduced into a host, so that the host is allowed to generate the antibody (EP 239400, International Publication WO96/02576, etc.).

In addition, a method of obtaining a human antibody has also been known. For example, human lymphocytes are sensitized with a desired antigen or a cell expressing the desired antigen *in vitro,* and then fusing the sensitized lymphocytes with human myeloma cells, such as, for example, U266, so as to obtain a desired human antibody having a binding activity to an antigen (JP Paten Publication (Kokoku) No. 1-59878 B (1989)). Otherwise, a transgenic antibody having all repertoires of human antibody genes is immunized with a desired antigen to obtain a desired human antibody (see WO93/12227, WO92/03918, WO94/02602, WO94/25585, WO96/34096, and WO96/33735). Further, a technique of obtaining a human antibody by panning using a human antibody library has also been known. For example, a human antibody variable region is allowed to express as a single chain antibody (scFv) on the surface of a phage according to a phage display method, and a phage binding to an antigen can be then selected. By analyzing the selected phage gene, a DNA sequence encoding the variable region of a human antibody binding to the antigen can be determined. If the DNA sequence of scFv binding to an antigen is clarified, a suitable expression vector comprising the sequence can be produced, so that a human antibody can be obtained. These methods have already been publicly known, and please refer to WO92/01047, WO92/20791, WO93/06213, WO93/11236, WO93/19172, WO95/01438, and WO95/15388.

The antibody that binds to tumor cells is preferably a humanized or a human antibody, but is not limited thereto.

Moreover, these antibodies may also be low molecular weight antibodies such as antibody fragments, or modified forms of the antibodies, unless they lose the property of recognizing the entire or a part of a protein encoded by an antigen gene present on the surface of tumor cells. The antibody fragment is a part of an antibody that retains a binding ability to ROBO1. Specific examples of the antibody fragment may include Fab, Fab', F(ab')2, Fv, Diabody, and a single chain variable fragment (scFv). In order to obtain such an antibody fragment, a gene encoding such an antibody fragment is constructed, the gene is then introduced into an expression vector, and it may be then expressed in suitable host cells. As a modified form of an antibody, an antibody binding to various types of molecules such as polyethylene glycol (PEG) can also be used.

DNA encoding a monoclonal antibody can be easily isolated and sequenced according to a commonly used method (for example, by using an oligonucleotide probe capable of specifically binding to a gene encoding the heavy chain and light chain of the monoclonal antibody). Hybridoma cells may be preferable starting materials for such DNA. Once such DNA is isolated, it is inserted into an expression vector, and the expression vector is then used to transform host cells such as *E. coli* cells, COS cells, CHO cells, or myeloma cells that do not generate immunoglobulin before they are transformed. Then, a monoclonal antibody can be generated from the transformed host cells.

### < Cytotoxin >

The cytotoxin is

### < Conjugate of Substance that Binds to Target Substance on Surface of Tumor Cells and Cytotoxin >

A substance that binds to a target substance on the surface of tumor cells, and a cytotoxin, must bind to each other, directly or indirectly.

When an antibody or a fragment thereof is used as such a substance that binds to a target substance on the surface of tumor cells, as a method of directly chemically binding the antibody or the fragment thereof to a cytotoxin, a binding method used for known ADC (Antibody Drug Conjugate) can be used.

Moreover, as another method, a method of indirectly binding an antibody or a fragment thereof to a cytotoxin by using a second binding pair can also be used. Examples of the second binding pair that can be utilized herein may include avidin-biotin and an antibody-hapten.

### < Administration Methods and Applied Doses >

The method for administering the medicament of the present invention to a subject having a tumor (for example, a cancer, etc.) is not particularly limited.

The conjugate of a substance that binds to a target substance on the surface of tumor cells and a cytotoxin can be administered to the subject, for example, via intravenous administration, arterial administration, intramuscular administration, subcutaneous administration, intradermal administration, intraperitoneal administration, or oral administration. Alternatively, an administration method involving administration of the conjugate to tumor tissues and the periphery thereof via local injection, application, spraying or the like can also be applied.

Talaporfin Sodium can be administered to a subject, for example, via intravenous administration, arterial administration, intramuscular administration, subcutaneous administration, intradermal administration, intraperitoneal administration, or oral administration. Alternatively, an administration method involving administration of the Talaporfin Sodium to tumor tissues and the periphery thereof via local injection, application, spraying or the like can also be applied.

The applied dose of the conjugate of a substance that binds to a target substance on the surface of tumor cells and a cytotoxin is not particularly limited. The conjugate can be administered to a subject at a dose of, for example, 1 µg/kg of body weight to 100 mg/kg of body weight, and preferably, at a dose of 10 µg/kg of body weight to 10 mg/kg of body weight.

The applied dose of the Talaporfin Sodium is not particularly limited. The Talaporfin Sodium can be administered to a subject at a dose of, for example, 1 µg/kg of body weight to 100 mg/kg of body weight, and preferably, at a dose of 10 µg/kg of body weight to 10 mg/kg of body weight.

The number of doses is not particularly limited, and administration can be carried out once to several times (from once to 20 times, and preferably from once to 10 times). Administration can be carried out, for example, every 2 to 4 weeks, or every 1 to 2 months. In addition, the number of light irradiation operations is not particularly limited, either. The light irradiation can be carried out once to several times.

### < Cells and/or Diseases as Targets >

The tumor as a target of the administration of the medicament of the present invention is a tumor that expresses Epidermal Growth Factor Receptor (EGFR, ERBB1, ERBB2, ERBB3, or ERBB4), Mesothelin, Ephrin type-A receptor 2 (EphA2), Glypican 3 (GPC3), Cadhelin 17 (CDH17), Cadhelin 3 (CDH3), Roundabout homolog 1 (Robo1) or the like on the surface thereof.

Specific examples of the tumor as a target of the administration of the medicament of the present invention may include cancers, such as head and neck cancer, lung cancer, liver cancer, colorectal cancer, skin cancer, esophageal cancer, stomach cancer, cervical cancer, endometrial cancer, mesothelioma, brain tumor, malignant melanoma, breast cancer, bile duct cancer, pancreatic cancer, ovarian cancer, kidney cancer, bladder cancer, prostate cancer, malignant lymphoma, and osteosarcoma.

Moreover, the present invention can be used in the treatment of animals other than humans, such as dogs, cats or horses, as well as the treatment of the diseases of humans.

### Examples

### Example 1:

### < Acquisition of Materials >

As an EGFR-expressing cell line, A431 (human epithelial-like cell carcinomaderived cell line) was acquired from KAC Company (Kyoto, Japan). As an anti-EGFR antibody, Cetuximab was acquired from Selleck Biotech, Ltd. (Tokyo, Japan). As a photosensitizer, Talaporfin Sodium was purchased from Medchemexpress (New Jersey) and was then used. An LED lamp (54 W) having a peak wavelength at 650 nm was purchased from King Do Way (18PCS E27, Amazon.co.jp).

### < Cell Culture >

Using a medium prepared by adding 10% fetal bovine serum to a high-glucose Dulbecco's Modified Eagle's Medium (DMEM), A431 was cultured under conditions of 37°C and 5% CO₂ concentration.

### < Adjustment of Immunotoxin >

Cetuximab dissolved in PBS(-) was mixed with EZ-LINK sulfo-NHS-LC-biotinylation reagent (Thermo Fisher Scientific, Commonwealth of Massachusetts) dissolved in ultrapure water at a molar ratio of 1 : 40, and the obtained mixture was then reacted. Thereafter, the reaction mixture was purified using PD SpinTrap G-25 (GE Healthcare Life Sciences, England). The biotinylated Cetuximab was equivalently mixed with streptavidin-saporin (Biotin-Z Internalization Kit [KIT-27-Z], Advanced Targeting Systems, California), and the obtained mixture was reacted at room temperature for 30 minutes, so as to obtain saporin-conjugated Cetuximab (IT-Cetuximab).

### < Cytotoxicity Assay >

A431 was seeded in a 96-well plate in an amount of 1.0 x 10⁴ cells per well, and was then cultured overnight. Subsequently, under the following three conditions, addition of an immunotoxin and a photosensitizer and light irradiation were carried out, and the degrees of cytotoxicity under individual conditions were compared with one another.
Condition 1: Addition of only 0.006 - 4 nM IT-Cetuximab
Condition 2: Addition of 0.006 - 4 nM IT-Cetuximab and 30 µM Talaporfin Sodium
Condition 3: Light irradiation (650 nm, 37.6 mJ/cm²), after addition of 0.006 - 4 nM IT-Cetuximab and 30 µM Talaporfin Sodium

IT-Cetuximab and Talaporfin Sodium were added to the cells under individual conditions, and 16 hours later, the cells were washed once using PBS(-). Thereafter, a novel drug-free medium was added to the resulting cells. Further, 4 hours later, the cell group under Condition 3 was irradiated with a light of 650 nm, so as to result in 37.6 mJ/cm².

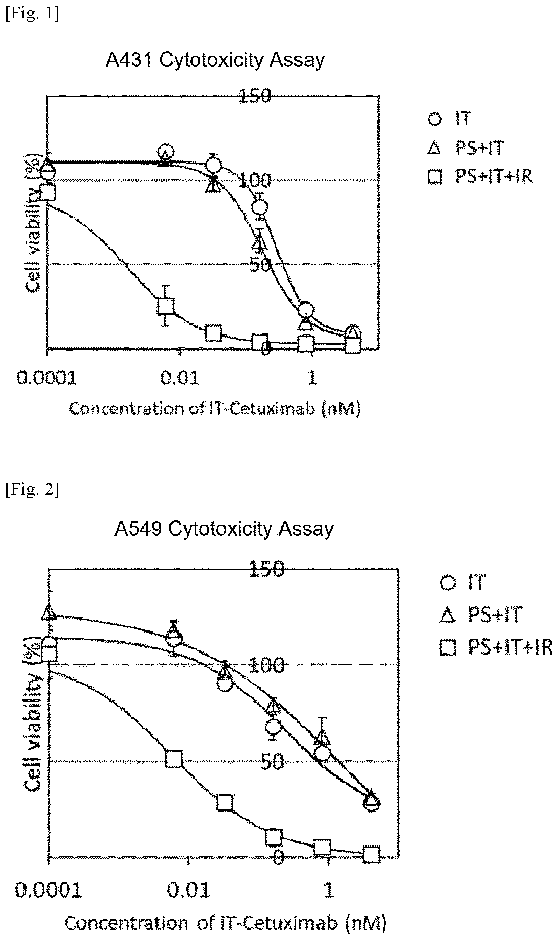

After the cells had been cultured for 72 hours, CCK-8 kit solution (Cell Counting Kit-8, DOJINDO LABORATORIES, Japan) was added in an amount of 10 µl to each well, and the reaction was then carried out for 1 to 2 hours. Thereafter, the absorption at 450 nm of each well was measured. Using the obtained results, cell viability was calculated according to the following calculating equation (Fig. 1): $Cell viability \left(%\right) = \left(a-c\right) / \left(b-c\right) \times 100 .$

In the above equation, "a" indicates the absorbance of each specimen, "b" indicates the absorbance of a negative control specimen not containing IT, and "c" indicates the absorbance of a medium alone.

Regarding the EC50 value, using the open source analysis software ImageJ, the mean results were fitted to a sigmoid curve, so as to obtain the IT-Cetuximab concentration showing 50% viability (Table 1).

### < Results >

IT-Cetuximab was confirmed to have concentration-dependent cytotoxic activity against the A431 cell line, and the 50% effective concentration (EC50) was found to be 0.36 nM. In addition, in the group in which IT-Cetuximab and Laserphyrin were simultaneously added to the cells under Condition 2, IT-Cetuximab was confirmed to have concentration-dependent cytotoxic activity, but the EC50 value was 0.23 nM, which was almost the same as the results obtained under Condition 1. Thus, the influence by addition of Laserphyrin was not observed. On the other hand, in the group in which IT-Cetuximab and Laserphyrin were simultaneously added to the cells and then light irradiation was carried out under Condition 3, the cytotoxic activity was significantly increased, and the EC50 value was 1.5 pM.

**[Table 1]**

| Table 1: EC50 of IT-Cetuximab against A431 | | |
|---|---|---|
| | Conditions | EC50 |
| Condition 1 | IT-Cetuximab | 0.36 nM |
| Condition 2 | IT-Cetuximab + Laserphyrin | 0.23 nM |
| Condition 3 | IT-Cetuximab + Laserphyrin + Light Irradiation | 1.5 pM |

### Example 2:

### < Acquisition of Materials >

As an EGFR-expressing cell line, A549 (human lung cancer cells) was acquired from KAC Company (Kyoto, Japan). With regard to Cetuximab used as an anti-EGFR antibody, Talaporfin Sodium used as a photosensitizer, and an LED lamp having a peak wavelength at 650 nm, the same as those used in Example 1 was used herein.

### < Cell Culture >

Using a medium prepared by adding 10% fetal bovine serum to a high-glucose Dulbecco's Modified Eagle's Medium (DMEM), A549 was cultured under conditions of 37°C and 5% CO₂ concentration.

### < Adjustment of Immunotoxin >

The same saporin-added Cetuximab (IT-Cetuximab) as that produced in Example 1 was used herein.

### < Cytotoxicity Assay >

A549 was seeded in a 96-well plate in an amount of 2.5 x 10³ cells per well, and was then cultured overnight. Subsequently, under the following three conditions, addition of an immunotoxin and a photosensitizer and light irradiation were carried out, and the degrees of cytotoxicity under individual conditions were compared with one another.
Condition 1: Addition of only 0.006 - 4 nM IT-Cetuximab
Condition 2: Addition of 0.006 - 4 nM IT-Cetuximab and 30 µM Talaporfin Sodium
Condition 3: Light irradiation (650 nm, 37.6 mJ/cm²), after addition of 0.006 - 4 nM IT-Cetuximab and 30 µM Talaporfin Sodium

IT-Cetuximab and Talaporfin Sodium were added to the cells under individual conditions, and 16 hours later, the cells were washed once using PBS(-). Thereafter, a novel drug-free medium was added to the resulting cells. Further, 4 hours later, the cell group under Condition 3 was irradiated with a light of 650 nm, so as to result in 37.6 mJ/cm².

After the cells had been cultured for 72 hours, the CCK-8 kit solution was added in an amount of 10 µl to each well, and the reaction was then carried out for 1 to 2 hours. Thereafter, the absorption at 450 nm of each well was measured. Based on the absorbance obtained in the same manner as that of Example 1, cell viability (Fig. 2) and the IT-Cetuximab concentration showing 50% viability were calculated (Table 2).

### < Results >

IT-Cetuximab was confirmed to have concentration-dependent cytotoxic activity against the A549 cell line, and the 50% effective concentration (EC50) was found to be 0.70 nM. In addition, in the group in which IT-Cetuximab and Laserphyrin were simultaneously added to the cells under Condition 2, IT-Cetuximab was confirmed to have concentration-dependent cytotoxic activity, but the EC50 value was 1.4 nM, which was almost the same as the results obtained under Condition 1. Thus, the influence by addition of Laserphyrin was not observed. On the other hand, in the group in which IT-Cetuximab and Laserphyrin were simultaneously added to the cells and then light irradiation was carried out under Condition 3, the cytotoxic activity was significantly increased, and the EC50 value was 6.9 pM.

**[Table 2]**

| Table 2: EC50 of IT-Cetuximab against A549 | | |
|---|---|---|
| | Conditions | EC50 |
| Condition 1 | IT-Cetuximab | 0.70 nM |
| Condition 2 | IT-Cetuximab + Laserphyrin | 1.4 nM |
| Condition 3 | IT-Cetuximab + Laserphyrin + Light Irradiation | 6.9 pM |

### Example 3: Cytotoxicity assay using HER2-expressing cell lines (MKN7 and MDA-MB-453), Talaporfin Sodium, and IT-Trastuzumab

### < Cell Culture >

The experiment was carried out using, as HER2-expressing cell lines, MKN7 (human stomach cancer-derived cells) and MDA-MB-453 (human breast cancer cells). MKN7 was cultured using a medium prepared by adding 10% fetal bovine serum to an RPMI1640 medium, under conditions of 37°C and 5% CO₂ concentration. On the other hand, MDA-MB-453 was cultured using a medium prepared by adding 10% fetal bovine serum to an L-15 medium, under conditions of 37°C and 0% CO₂ concentration.

### < Adjustment of Immunotoxin >

As an anti-HER2 antibody, Trastuzumab (Selleck Biotech, Ltd.) was used, and saporin-added Trastuzumab (IT-Trastuzumab) was obtained by the same method as the method of adjusting an immunotoxin applied in Example 1.

### < Cytotoxicity Assay >

MKN7 and MDA-MB-453 were each seeded in a 96-well plate in an amount of 1 x 10⁴ cells per well, and were then cultured overnight. Subsequently, under the following three conditions, addition of an immunotoxin and a photosensitizer and light irradiation were carried out, and the degrees of cytotoxicity under individual conditions were compared with one another.
Condition 1: Addition of only 0.032 - 20 nM IT-Trastuzumab
Condition 2: Addition of 0.032 - 20 nM IT-Trastuzumab and 20 µM Talaporfin Sodium
Condition 3: Light irradiation (650 nm, 37.6 mJ/cm²), after addition of 0.032 - 20 nM IT-Trastuzumab and 20 µM Talaporfin Sodium

IT-Trastuzumab and Talaporfin Sodium were added to the cells under individual conditions, and 16 hours later, the cells were washed once with PBS(-). Then, a novel drug-free medium was added to the resulting cells. Further, 4 hours later, the cell group under Condition 3 was irradiated with a light of 650 nm, so as to result in 37.6 mJ/cm².

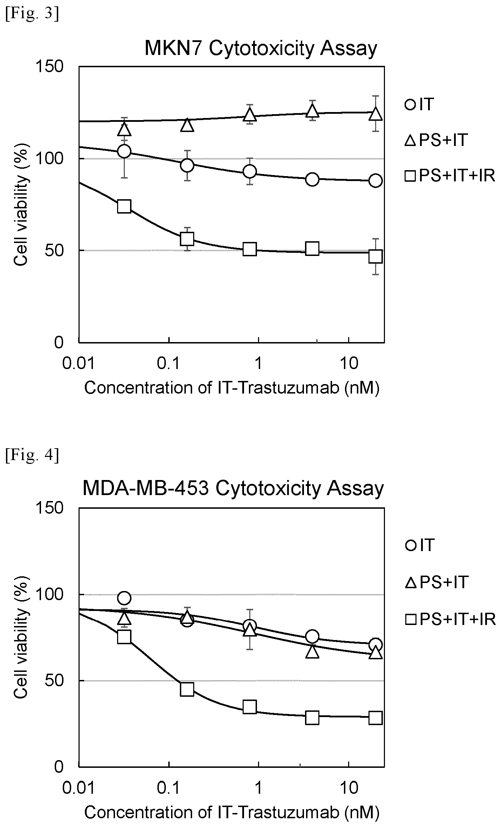

The cells were cultured for 72 hours, and thereafter, the CCK-8 kit solution was added in an amount of 10 µl to each well, and the reaction was then carried out for 1 to 2 hours. Thereafter, the absorption at 450 nm of each well was measured. Based on the absorbance obtained in the same manner as that of Example 1, cell viability (Fig. 3 and Fig. 4) and the IT-Cetuximab concentration showing 50% viability were calculated.

### < Results >

In the cell group in which only IT-Trastuzumab was added to the MKN7 cell line, almost no cytotoxic activity was observed in the concentration range (20 nM or less) of IT-Trastuzumab added this time. Likewise, also in the group in which IT-Cetuximab and Laserphyrin were simultaneously added to the cells under Condition 2, cytotoxic activity was not observed. Thus, the influence by addition of Laserphyrin was not observed. On the other hand, in the group in which IT-Cetuximab and Laserphyrin were added to the cells and then light irradiation was carried out under Condition 3, the cytotoxic activity was observed in a concentration-dependent manner, and the EC50 value was 1.01 nM. Such concentration-dependent cytotoxic activity against the MDA-MB-453 cell line was observed only under Condition 3, and the EC50 value was 0.12 nM.

### Example 4: Cytotoxicity assay using EGFR-expressing cell line (A431), Talaporfin Sodium, and peptide toxin (PEP-T)

### < Cell Culture >

The experiment was carried out using A431 as an EGFR-expressing cell line. As in the same manner as that of Example 1, A431 was cultured using a medium prepared by adding 10% fetal bovine serum to a high-glucose Dulbecco's Modified Eagle's Medium (DMEM), under conditions of 37°C and 5% CO₂ concentration.

### < Adjustment of Peptide Toxin >

A peptide molecule has been known as a substance that binds to EGFR. Examples of such a peptide molecule may include: peptide GE11 (sequence: YHWYGYTPQNVI) identified in 2005 from a phage display peptide library [Li, Z. et al. Identification and characterization of a novel peptide ligand of epidermal growth factor receptor for targeted delivery of therapeutics. FASEB J. 2005, 19, 1978-1985.]; and 6-mer peptide D4 (sequence: LARLLT) discovered by computer designing [Song, S. et al. Novel peptide ligand directs liposomes toward EGF-R high-expressing cancer cells in vitro and in vivo. FASEB J. 2009, 23, 1396-1404.]. In the present invention, was used a peptide (sequence: YHWYGYTPENVI) that is an analog of GE11 and is found to bind to EGFR.

The aforementioned peptide, which had a biotinylated C-terminus, was purchased from COSMO BIO COMPANY, LIMITED, and was used in the experiment. The purchased biotin peptide was equivalently mixed with streptavidin-saporin, and the obtained mixture was then reacted at room temperature for 30 minutes, so as to obtain a saporin-added peptide (PEP-T).

### < Cytotoxicity Assay >

A431 was seeded in a 96-well plate in an amount of 1 x 10⁴ cells per well, and was then cultured overnight. Subsequently, under the following three conditions, addition of PEP-T and a photosensitizer and light irradiation were carried out, and the degrees of cytotoxicity under individual conditions were compared with one another.
Condition 1: Addition of only 0.032 - 20 nM PEP-T
Condition 2: Addition of 0.032 - 20 nM PEP-T and 20 µM Talaporfin Sodium
Condition 3: Light irradiation (650 nm, 37.6 mJ/cm²), after addition of 0.032 - 20 nM PEP-T and 20 µM Talaporfin Sodium

PEP-T and Talaporfin Sodium were added to the cells under individual conditions, and 16 hours later, the cells were washed once using PBS(-). Thereafter, a novel drug-free medium was added to the resulting cells. Further, 4 hours later, the cell group under Condition 3 was irradiated with a light of 650 nm, so as to result in 37.6 mJ/cm².

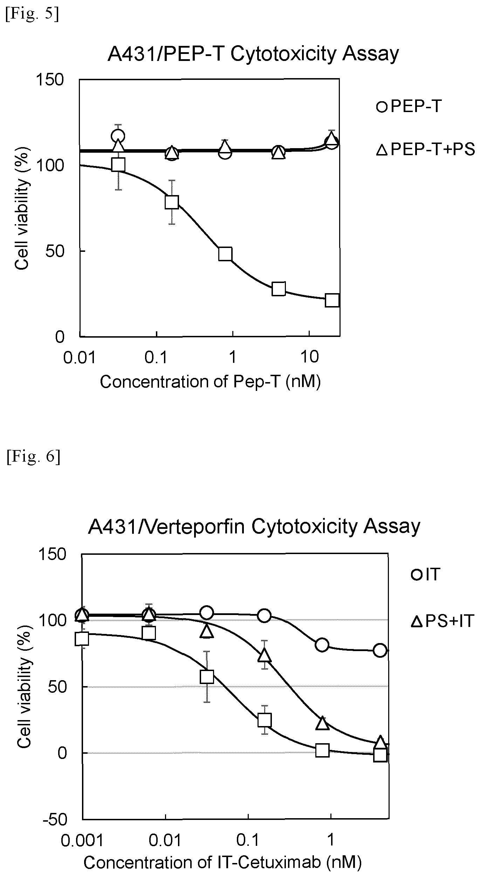

After the cells had been cultured for 72 hours, the CCK-8 kit solution was added in an amount of 10 µl to each well, and the reaction was then carried out for 1 to 2 hours. Thereafter, the absorption at 450 nm of each well was measured. Based on the absorbance obtained in the same manner as that of Example 1, cell viability (Fig. 5) and the PEP-T concentration showing 50% viability were calculated.

### < Results >

In the group in which only PEP-T was added to the A431 cell line, almost no cytotoxic activity was observed in the concentration range (20 nM or less) of PEP-T added this time. Likewise, also in the group in which PEP-T and Laserphyrin were simultaneously added to the cells under Condition 2, cytotoxic activity was not observed. Thus, the influence by addition of Laserphyrin was not observed. On the other hand, in the group in which PEP-T and Laserphyrin were added to the cells and then light irradiation was carried out under Condition 3, the cytotoxic activity was observed in a concentration-dependent manner, and the EC50 value was 0.70 nM.

### Example 6: Cytotoxicity assay using EGFR-expressing cell line (A431), Verteporfin, and IT-Cetuximab

### < Acquisition of Materials >

Verteporfin used as a photosensitizer was acquired from Cayman Chemical (U.S.A.).

With regard to A431 used an EGFR-expressing cell line, Cetuximab used as an anti-EGFR antibody, and an LED lamp having a peak wavelength at 650 nm, the same as those used in Example 1 was used herein.

### < Cell Culture >

A431 was cultured using a medium prepared by adding 10% fetal bovine serum to a high-glucose Dulbecco's Modified Eagle's Medium (DMEM), under conditions of 37°C and 5% CO₂ concentration.

### < Adjustment of Immunotoxin >

The same saporin-added Cetuximab (IT-Cetuximab) as that produced in Example 1 was used herein.

### < Cytotoxicity Assay >

A431 was seeded in a 96-well plate in an amount of 1 x 10⁴ cells per well, and was then cultured overnight. Subsequently, under the following three conditions, addition of an immunotoxin and a photosensitizer and light irradiation were carried out, and the degrees of cytotoxicity under individual conditions were compared with one another.
Condition 1: Addition of only 0.006 - 4 nM IT-Cetuximab
Condition 2: Addition of 0.006 - 4 nM IT-Cetuximab and 0.7 µM Verteporfin
Condition 3: Light irradiation (650 nm, 2.82 J/cm²), after addition of 0.006 - 4 nM IT-Cetuximab and 0.7 µM Verteporfin

Under individual conditions, IT-Cetuximab was added to the cells, and 20 hours after addition of IT-Cetuximab, Verteporfin was added to the reaction mixture. Further, 2 hours after addition of Verteporfin, the resulting cells were washed once using PBS(-), and a novel drug-free medium was added thereto. The cell group under Condition 3 was irradiated with a light of 650 nm, so as to result in 2.82 J/cm².

After the cells had been cultured for 72 hours, the CCK-8 kit solution was added in an amount of 10 µl to each well, and the reaction was then carried out for 1 to 2 hours. Thereafter, the absorption at 450 nm of each well was measured. Based on the absorbance obtained in the same manner as that of Example 1, cell viability (Fig. 6) and the IT-Cetuximab concentration showing 50% viability were calculated.

### < Results >

It was observed that IT-Cetuximab exhibited concentration-dependent cytotoxic activity against the A431 cell line. In addition, in the group in which IT-Cetuximab and Verteporfin were simultaneously added to the cells under Condition 2, the cytotoxic activity of IT-Cetuximab was observed in a concentration-dependent manner, and the EC50 value was 0.3 nM. Compared with addition of IT-Cetuximab alone, the cytotoxic activity tended to be increased by addition of Verteporfin. Moreover, under Condition 3 in which IT-Cetuximab and Verteporfin were added and light irradiation was then carried out, the cytotoxic activity was further increased, and the EC50 value was 0.05 nM.

## Claims

1. A medicament for use in a method of killing tumor cells in a subject, comprising:
a conjugate of saporin and an antibody or an antibody fragment that binds to a target substance on the surface of tumor cells; and
Talaporfin Sodium,
wherein the medicament is for use in combination with irradiation of wavelength effective for activating the Talaporfin Sodium.

2. The medicament for use according to claim 1, wherein the tumor cells are cells that express Epidermal Growth Factor Receptor (EGFR, ERBB1, ERBB2, ERBB3, or ERBB4), Mesothelin, Ephrin type-A receptor 2 (EphA2), Glypican 3 (GPC3), Cadherin 17 (CDH17), or Roundabout homolog 1 (Robo1) on the cell surface thereof.

3. The medicament for use according to any one of claims 1 or 2, wherein the tumor cells are cancer cells of any one of head and neck cancer, lung cancer, liver cancer, colorectal cancer, skin cancer, esophageal cancer, stomach cancer, cervical cancer, endometrial cancer, mesothelioma, brain tumor, malignant melanoma, breast cancer, bile duct cancer, pancreatic cancer, ovarian cancer, kidney cancer, bladder cancer, prostate cancer, malignant lymphoma, and osteosarcoma.

4. The medicament for use according to any one of claims 1 to 3, which kills tumor cells by performing the following steps on the cells:
(1) a step of allowing the conjugate to come into contact with tumor cells;
(2) a step of allowing Talaporfin Sodium to come into contact with the tumor cells after step (1); and
(3) a step of irradiating the tumor cells with a wavelength effective for activating the Talaporfin Sodium, so as to kill the cells, after step (2).

5. The medicament for use according to any one of claims 1 to 3, which kills tumor cells by performing the following steps on the cells:
(1) a step of allowing Talaporfin Sodium to come into contact with tumor cells
(2) a step of allowing the conjugate to come into contact with the tumor cells after step (1); and
(3) a step of irradiating the tumor cells with a wavelength effective for activating the Talaporfin Sodium so as to kill the cells after step (2).

6. The medicament for use according to any one of claims 1 to 3, which kills tumor cells by performing the following steps on the cells:
(1) a step of allowing the conjugate and Talaporfin Sodium to come into contact with tumor cells; and then,
(2) a step of irradiating the tumor cells with a wavelength effective for activating the Talaporfin Sodium so as to kill the cells.

7. The medicament for use according to any one of claims 1 to 6, wherein the substance that binds to a target substance on the surface of tumor cells is anti-EGFR antibody, the cytotoxin is saporin, and the medicament comprises Talaporfin Sodium.

8. The medicament for use according to claim 7, wherein the anti-EGFR antibody is Cetuximab.

9. The medicament for use according to any one of claims 1 to 6, wherein the substance that binds to a target substance on the surface of tumor cells is anti-HER2 antibody, the cytotoxin is saporin, and the medicament comprises Talaporfin Sodium.

10. The medicament for use according to claim 9, wherein the anti-HER2 antibody is Trastuzumab.

## Patentansprüche

1. Medikament zur Verwendung in einem Verfahren zum Abtöten von Tumorzellen in einem Subjekt, umfassend:
ein Konjugat aus Saporin und einem Antikörper oder einem Antikörperfragment, der bzw. das an eine Zielsubstanz auf der Oberfläche von Tumorzellen bindet; und
Talaporfin-Natrium,
wobei das Medikament zur Verwendung in Kombination mit Bestrahlung mit einer zur Aktivierung des Talaporfin-Natriums wirksamen Wellenlänge ist.

2. Das Medikament zur Verwendung gemäß Anspruch 1, wobei die Tumorzellen Zellen sind, die epidermalen Wachstumsfaktorrezeptor (EGFR, ERBB1, ERBB2, ERBB3 oder ERBB4), Mesothelin, Ephrin-Typ-A-Rezeptor 2 (EphA2), Glypican 3 (GPC3), Cadherin 17 (CDH17) oder Roundabout-Homolog 1 (Robo1) auf ihrer Zelloberfläche exprimieren.

3. Das Medikament zur Verwendung gemäß einem der Ansprüche 1 oder 2, wobei die Tumorzellen eine der folgenden Krebszellen sind: Kopf-Hals-Krebs, Lungenkrebs, Leberkrebs, Darmkrebs, Hautkrebs, Speiseröhrenkrebs, Magenkrebs, Gebärmutterhalskrebs, Gebärmutterschleimhautkrebs, Mesotheliom, Hirntumor, malignes Melanom, Brustkrebs, Gallenwegskrebs, Bauchspeicheldrüsenkrebs, Eierstockkrebs, Nierenkrebs, Blasenkrebs, Prostatakrebs, malignes Lymphom und Osteosarkom.

4. Das Medikament zur Verwendung gemäß einem der Ansprüche 1 bis 3, das Tumorzellen abtötet, indem die folgenden Schritte an den Zellen durchgeführt werden:
(1) ein Schritt, bei dem das Konjugat mit den Tumorzellen in Kontakt kommen gelassen wird;
(2) ein Schritt, bei dem Talaporfin-Natrium mit den Tumorzellen in Kontakt kommen gelassen wird, nach Schritt (1); und
(3) ein Schritt, bei dem die Tumorzellen mit einer Wellenlänge, die zur Aktivierung des Talaporfin-Natriums wirksam ist, bestrahlt werden, um die Zellen abzutöten, nach Schritt (2).

5. Das Medikament zur Verwendung gemäß einem der Ansprüche 1 bis 3, das Tumorzellen abtötet, indem die folgenden Schritte an den Zellen durchgeführt werden:
(1) ein Schritt, bei dem Talaporfin-Natrium mit den Tumorzellen in Kontakt kommen gelassen wird;
(2) ein Schritt, bei dem das Konjugat mit den Tumorzellen in Kontakt kommen gelassen wird, nach Schritt (1); und
(3) ein Schritt, bei dem die Tumorzellen mit einer Wellenlänge, die zur Aktivierung des Talaporfin-Natriums wirksam ist, bestrahlt werden, um die Zellen abzutöten, nach Schritt (2).

6. Das Medikament zur Verwendung gemäß einem der Ansprüche 1 bis 3, das Tumorzellen abtötet, indem die folgenden Schritte an den Zellen durchgeführt werden:
(1) ein Schritt, bei dem das Konjugat und Talaporfin-Natrium mit den Tumorzellen in Kontakt kommen gelassen wird; und anschließend
(2) ein Schritt, bei dem die Tumorzellen mit einer Wellenlänge, die zur Aktivierung des Talaporfin-Natriums wirksam ist, bestrahlt werden, um die Zellen abzutöten.

7. Das Medikament zur Verwendung gemäß einem der Ansprüche 1 bis 6, wobei die Substanz, die an eine Zielsubstanz auf der Oberfläche von Tumorzellen bindet, ein Anti-EGFR-Antikörper ist, das Zytotoxin Saporin ist und das Medikament Talaporfin-Natrium umfasst.

8. Das Medikament zur Verwendung gemäß Anspruch 7, wobei der Anti-EGFR-Antikörper Cetuximab ist.

9. Das Medikament zur Verwendung gemäß einem der Ansprüche 1 bis 6, wobei die Substanz, die an eine Zielsubstanz auf der Oberfläche von Tumorzellen bindet, ein Anti-HER2-Antikörper ist, das Zytotoxin Saporin ist und das Medikament Talaporfin-Natrium umfasst.

10. Das Medikament zur Verwendung gemäß Anspruch 9, wobei der Anti-HER2-Antikörper Trastuzumab ist.

## Revendications

1. Médicament destiné à être utilisé dans un procédé de destruction de cellules tumorales chez un sujet, comprenant :
un conjugué de saporine et d'un anticorps ou d'un fragment d'anticorps qui se lie à une substance cible à la surface des cellules tumorales ; et
de la talaporfine sodique,
dans lequel le médicament est destiné à être utilisé en combinaison avec une irradiation de longueur d'onde efficace pour activer la talaporfine sodique.

2. Médicament destiné à être utilisé selon la revendication 1, dans lequel les cellules tumorales sont des cellules qui expriment le récepteur du facteur de croissance épidermique (EGFR, ERBB1, ERBB2, ERBB3, ou ERBB4), la mésothéline, le récepteur 2 de type A des éphrines (EphA2), la glypicane 3 (GPC3), la cadhérine 17 (CDH17), ou l'homologue 1 de Roundabout (Robo1) à leur surface cellulaire.

3. Médicament destiné à être utilisé selon l'une quelconque des revendications 1 ou 2, dans lequel les cellules tumorales sont des cellules cancéreuses de l'un quelconque parmi le cancer de la tête et du cou, le cancer du poumon, le cancer du foie, le cancer colorectal, le cancer de la peau, le cancer de l'œsophage, le cancer de l'estomac, le cancer du col de l'utérus, le cancer de l'endomètre, le mésothéliome, la tumeur cérébrale, le mélanome malin, le cancer du sein, le cancer des voies biliaires, le cancer du pancréas, le cancer de l'ovaire, le cancer du rein, le cancer de la vessie, le cancer de la prostate, le lymphome malin et l'ostéosarcome.

4. Médicament destiné à être utilisé selon l'une quelconque des revendications 1 à 3, qui détruit des cellules tumorales par la mise en œuvre sur les cellules des étapes suivantes :
(1) une étape consistant à mettre le conjugué en contact avec des cellules tumorales ;
(2) une étape consistant à mettre la talaporfine sodique en contact avec les cellules tumorales après l'étape (1) ; et
(3) une étape consistant à irradier les cellules tumorales avec une longueur d'onde efficace pour activer la talaporfine sodique, de manière à détruire les cellules, après l'étape (2).

5. Médicament destiné à être utilisé selon l'une quelconque des revendications 1 à 3, qui détruit des cellules tumorales par l'exécution sur les cellules des étapes suivantes :
(1) une étape consistant à mettre la talaporfine sodique en contact avec des cellules tumorales
(2) une étape consistant à mettre le conjugué en contact avec les cellules tumorales après l'étape (1) ; et
(3) une étape consistant à irradier les cellules tumorales avec une longueur d'onde efficace pour activer la talaporfine sodique de manière à détruire les cellules après l'étape (2).

6. Médicament destiné à être utilisé selon l'une quelconque des revendications 1 à 3, qui détruit des cellules tumorales par l'exécution sur les cellules des étapes suivantes :
(1) une étape consistant à mettre le conjugué et la talaporfine sodique en contact avec des cellules tumorales ; et ensuite,
(2) une étape consistant à irradier les cellules tumorales avec une longueur d'onde efficace pour activer la talaporfine sodique de manière à détruire les cellules.

7. Médicament destiné à être utilisé selon l'une quelconque des revendications 1 à 6, dans lequel la substance qui se lie à une substance cible à la surface des cellules tumorales est un anticorps anti-EGFR, la cytotoxine est la saporine, et le médicament comprend la talaporfine sodique.

8. Médicament destiné à être utilisé selon la revendication 7, dans lequel l'anticorps anti-EGFR est le cétuximab.

9. Médicament destiné à être utilisé selon l'une quelconque des revendications 1 à 6, dans lequel la substance qui se lie à une substance cible à la surface des cellules tumorales est un anticorps anti-HER2, la cytotoxine est la saporine, et le médicament comprend de la talaporfine sodique.

10. Médicament destiné à être utilisé selon la revendication 9, dans lequel l'anticorps anti-HER2 est le trastuzumab.
